(19) Europäisches Patentamt / European Patent Office / Office européen des brevets

(11) **EP 4 667 213 A1**

(12) **EUROPEAN PATENT APPLICATION**
published in accordance with Art. 153(4) EPC

(43) Date of publication:
**24.12.2025 Bulletin 2025/52**

(21) Application number: **24778938.1**

(22) Date of filing: **26.02.2024**

(51) International Patent Classification (IPC):
**B32B 7/022** (2019.01)   **A61F 13/02** (2024.01)
**A61F 13/15** (2006.01)   **A61F 13/49** (2006.01)
**B32B 27/20** (2006.01)   **B32B 27/32** (2006.01)
**C08J 7/04** (2020.01)

(52) Cooperative Patent Classification (CPC):
**A61F 13/02; A61F 13/15; A61F 13/49; B32B 7/022; B32B 27/20; B32B 27/32; C08J 7/04**

(86) International application number:
**PCT/JP2024/006828**

(87) International publication number:
**WO 2024/202796 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:  **29.03.2023  JP 2023052797**

(71) Applicant: **C.I. TAKIRON Corporation**
**Kita-Ku**
**Osaka-Shi**
**Osaka 530-0001 (JP)**

(72) Inventors:
• **MORI, Keiichi**
  **Osaka-shi, Osaka 530-0001 (JP)**
• **TANIGAWA, Yuhei**
  **Osaka-shi, Osaka 530-0001 (JP)**

(74) Representative: **advotec.**
**Patent- und Rechtsanwaltspartnerschaft mbB**
**Widenmayerstraße 4**
**80538 München (DE)**

(54)   **STRETCH FILM**

(57)   A stretchable film (1) includes: an elastomer layer (5); and a surface layer (6, 7) stacked on the surface of the elastomer layer (5). The elastomer layer (5) contains a styrene-based elastomer. The surface layer (6, 7) is made of an olefin-based resin composition containing an olefin-based resin and an inorganic filler. The stretchable film has a hysteresis loss of less than 36%.

FIG.1

## EP 4 667 213 A1

**Description**

TECHNICAL FIELD

The present invention relates to a stretchable film.

BACKGROUND ART

Stretchable films are used in a wide range of fields such as sanitary goods, sporting goods, and medical goods to improve handleability, wearing feeling (fitting feeling), and the like. For example, stretchable films are used for clothes such as underwear, a waistband of paper diapers, a side panel, a leg gather, an incontinence article, a sanitary napkin, a bandage, a surgical drape, a fastening band, a hat, swimming pants, a sports supporter, a medical supporter, and an adhesive plaster.

In stretchable films using elastomers having stretchability, there was a problem in that strong tackiness caused by the elastomers made handling difficult, especially for thin products such as films.

Accordingly, to prevent such tackiness caused by the elastomers, a stretchable film has been proposed that includes a surface layer formed of a resin with low tackiness on the surface of the elastomer. More specifically, for example, a stretchable film has been proposed that includes an elastomer layer and an olefin-based resin layer stacked on at least one surface of the elastomer layer (see, for example, Patent Document 1).

CITATION LIST

PATENT DOCUMENT

Patent Document 1: Japanese Unexamined Patent Publication No. 2016-112878

SUMMARY OF THE INVENTION

TECHNICAL PROBLEM

In general, the stretchability of film is evaluated based on its permanent strain (i.e., the strain that remains after an external force is applied to and then removed from the film). However, there has been a problem in that it is difficult to obtain a stretchable film having stretchability approximating that of an ideal elastic body, such as rubber, solely by relying on evaluation based on the permanent strain.

The present invention was made in view of the above problem, and an object of the present invention is to provide a stretchable film including an elastomer layer and a surface layer stacked on the surface of the elastomer layer and having excellent stretchability.

SOLUTION TO THE PROBLEM

In order to achieve the above object, a stretchable film of the present invention includes: an elastomer layer; and a surface layer stacked on at least one surface of the elastomer layer. The elastomer layer contains a styrene-based elastomer. The surface layer is made of an olefin-based resin composition containing an olefin-based resin and an inorganic filler. The stretchable film has a hysteresis loss of less than 36%.

(Hysteresis Loss)

A strip-shaped test piece is cut from the stretchable film. The test piece has 100 mm in one direction of the film and 25 mm in a direction orthogonal to the one direction. The test piece is then fixed to grippers of a tester so that a distance between the grippers is 25 mm, and the test piece is then elongated in its longitudinal direction at a speed 254 mm/min so that a strain (elongation) calculated by the following equation (1) is 100%, an integral area in a stress-strain curve (S-S curve) during the elongation is defined as S1, an integral area in a stress-strain curve (S-S curve) during contraction of the test piece at the same speed as for the elongation, immediately after the elongation so that s load (N/25 mm) of the test piece reaches 0 is defined as S2, and the hysteresis loss [%] is calculated from the following equation (2).

$$\text{Strain [\%]} = (L1 - L0)/L0 \times 100 \quad (1)$$

2

$$\text{Hysteresis loss } [\%] = S/S1 \times 100 \quad (2)$$

L0 is a distance (mm) between the grippers before the elongation, and L1 is a distance (mm) between the grippers after the elongation. S1 is an integral area in a stress-strain curve during elongation from 0% to 100% strain; S2 is an integral area in a stress-strain curve during contraction from 100% strain to 0 stress; and S is an integral area (S1 - S2) in a stress-strain curve obtained by subtracting the integral area S2 from the integral area S1.

ADVANTAGES OF THE INVENTION

The present invention makes it possible to provide a stretchable film including an elastomer layer and a surface layer stacked on the surface of the elastomer layer and having excellent stretchability.

BRIEF DESCRIPTION OF THE DRAWINGS

[FIG. 1] A sectional view of a stretchable film according to a first embodiment of the present invention.
[FIG. 2] A plan view of the stretchable film according to the first embodiment of the present invention.
[FIG. 3] A graph illustrating an integral area S1 of a stress-strain curve during elongation from 0% to 100% strain.
[FIG. 4] A graph illustrating an integral area S2 of a stress-strain curve during contraction from 100% strain to 0 stress.
[FIG. 5] A graph illustrating an integral area of a stress-strain curve obtained by subtracting the integral area S2 from the integral area S1.
[FIG. 6] A sectional view of a stretchable film according to a second embodiment of the present invention.
[FIG. 7] A plan view of the stretchable film according to the second embodiment of the present invention.

DESCRIPTION OF EMBODIMENTS

The stretchable film according to the present invention will be described in detail below. It should be noted that the present invention is not limited to the following embodiments and can be appropriately modified and applied without departing from the scope of the present invention.

[First Embodiment]

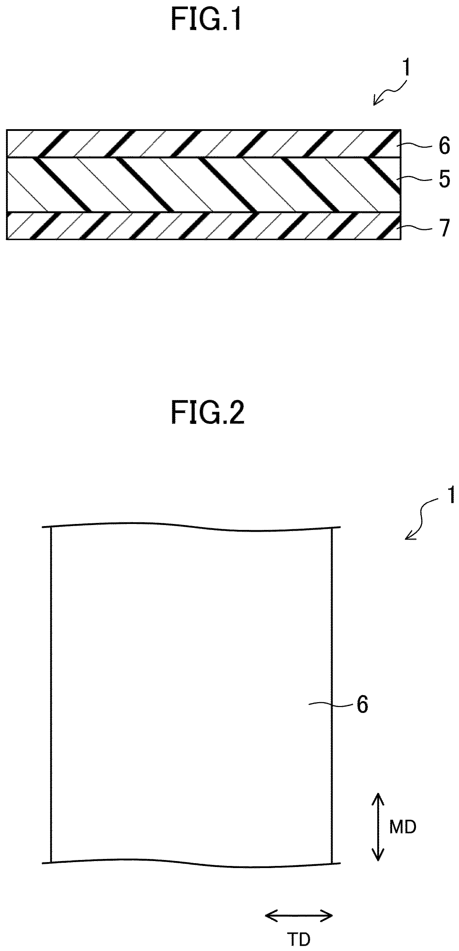

FIG. 1 is a sectional view of a stretchable film according to the present embodiment. As illustrated in FIG. 1, the stretchable film 1 of this embodiment includes an elastomer layer 5, and surface layers 6, 7 stacked on surfaces of the elastomer layer 5.

(Elastomer Layer)

The elastomer layer 5 is a layer that imparts stretchability to the stretchable film 1, and contains a styrene-based elastomer that is a thermoplastic elastomer.

<Thermoplastic Elastomer>

"Thermoplastic elastomer" refers to a polymer or a polymer blend that exhibit properties similar to those of vulcanized rubber at its use temperature, but loses these properties at a processing temperature, thereby allowing for easy processing, and exhibit again these properties when returned to the use temperature.
The use of the styrene-based elastomer makes the film less stretchable, thereby enhancing its dimensional stability during transport.
Examples of the styrene-based elastomer used in the present invention include a styrene-isoprene-styrene copolymer (SIS elastomer), a styrene-isoprene block copolymer, a styrene-butadiene-styrene block copolymer (SBS elastomer), a styrene-butadiene block copolymer, a hydrogenated styrene-isoprene-styrene block copolymer (styrene-ethylene-propylene-styrene block copolymer (SEPS elastomer)), a hydrogenated styrene-butadiene-styrene block copolymer (styrene-ethylene-butylene-styrene block copolymer (SEBS elastomer)), and a styrene-ethylene-ethylene-propylene-styrene copolymer (SEEPS elastomer), etc. Among these, the SIS elastomer is preferable because of having higher stretchability. One kind of these styrene-based elastomers may be used alone, or two or more kinds of them may be used in combination.

The thermoplastic elastomer generally includes hard segments that govern basic physical properties such as mechanical properties and soft segments that govern stretchability which is rubber-like properties. Examples of the hard segments of the styrene-based elastomer include polystyrene, and examples of the soft segments of the styrene-based elastomer include polybutadiene, polyisoprene, polyethylene, and hydrogenated products thereof.

When the SIS elastomer is used, the styrene unit content rate relative to the total units of the SIS elastomer is preferably 9 mass% or more and less than 50 mass%, preferably 12 mass% or more and less than 30 mass%. When the styrene unit content rate, which is the content rate of the hard segments is 9 mass% or more, the stress [N/mm$^2$] at 10% elongation in the machine axis (longitudinal) direction (hereinafter also referred to as "MD") of the stretchable film shown in FIG. 2 improves, thereby obtaining excellent transportability. When the styrene unit content is less than 50 mass%, excellent stretchability can be obtained in the direction orthogonal to the machine axis direction shown in FIG. 2 (hereinafter also referred to as "TD"), due to elasticity of the soft segments.

The Shore A hardness of the SIS elastomer is preferably 20 or more and less than 70. The Shore A hardness of 20 or more allows improvement in stress [N/mm$^2$] at 10% elongation in the MD. The Shore A hardness of less than 70 allows improvement in stretchability in the TD.

The "Shore A hardness" as used herein refers to a hardness measured using a type A durometer in accordance with JIS K 6253 at a temperature of 23°C.

When the SIS elastomer is used alone, the styrene unit content rate relative to the total units of the SIS elastomer (i.e., the entire elastomer layer) is preferably 30 mass% or less, more preferably 20 mass% or less. Since styrene has poor stretchability, when the styrene unit content rate of the SIS elastomer is more than 30 mass%, the hysteresis loss to be described later increases, and the stretchability of the stretchable film may decrease.

When the SIS elastomer is used in combination of other elastomers (for example, SIS elastomers having different styrene unit content rates are blended), the styrene unit content rate relative to the entire elastomer layer is preferably from 9 mass% to 35 mass% inclusive. When the styrene unit content rate is 9 mass% or more, the stress [N/mm$^2$] at 10% elongation in the MD improves, thereby obtaining excellent transportability, and when the styrene unit content rate is 35 mass% or less, excellent stretchability is obtained in the TD due to elasticity of the soft segments.

When the SIS elastomer is used, the Shore A hardness of the entire elastomer layer is preferably 30 or more and less than 60. The Shore A hardness of 30 or more allows improvement in the stress [N/mm$^2$] at 10% elongation in the MD. The Shore A hardness of less than 60 allows suppression of decrease in stretchability in the TD.

It was found that when the Shore A hardness of the entire elastomer layer was 30 or more, the stress [N/mm$^2$] of the stretchable film at 10% elongation in the MD improved, and excellent transportability was obtained, but stretchability in the TD decreased. That is, based on the fact that the transportability in the MD and the stretchability in the TD are in a trade-off relationship, the present inventors have found that the Shore A hardness of the entire elastomer layer is set to 30 or more to improve the stress [N/mm$^2$] at 10% elongation in the MD, and the styrene unit content rate of the entire elastomer layer is set to 35 mass% or less in order to improve the decreased stretchability in the TD.

The melt mass flow rate (MFR) of the styrene-based elastomer is preferably 0.5 g/10 min to 22 g/10 min, more preferably 3 g/10 min to 15 g/10 min. The MFR of the styrene-based elastomer exceeding 22 g/10 min may result in excessively low melt viscosity, which can make thickness control during film molding difficult. When the MFR of the styrene-based elastomer is less than 0.5 g/10 min, melt fracture may occur during film molding, which may result in a rough film surface, and the film may break during high-speed extrusion molding in the film molding because of the high melt viscosity.

The melt mass flow rate described above can be obtained by measurement in accordance with JIS K 7210.

The density of the styrene-based elastomer is preferably less than 0.99 g/cm$^3$. The density of the styrene-based elastomer being 0.99 g/cm$^3$ or more may cause a decrease in stretchability of the film.

<Other Components>

The elastomer layer 5 may contain another component besides the styrene-based elastomer described above as long as the effect of the present invention is not impaired. Examples of the other components include other resins than the styrene-based elastomer such as an olefin-based resin, an amide-based anti-blocking agent (such as octadecanamide), a plasticizer, an ultraviolet absorber, an antioxidant, a weathering agent, an antistatic agent, a colorant, an anti-fogging agent, metal soap, wax, an antifungal agent, an antibacterial agent, a nucleating agent, a flame retardant, a lubricant, etc. These other components may be added to the material for the stretchable film in the form of masterbatch.

(Surface Layer)

The surface layers 6, 7 are layers for reducing tackiness of the elastomer layer 5 and for suppressing blocking in the stretchable film 1. The surface layers are provided on one or both of the first surface and the second surface of the elastomer layer, but are provided preferably on both of the first surface and the second surface of the elastomer layer 5 as illustrated in FIG. 1 in light of sufficiently suppressing blocking in the stretchable film 1. The surface layers 6, 7 may be surface layers of the same type or different types.

Each of the surface layers 6, 7 may be made of an olefin-based resin composition containing an olefin-based resin and an inorganic filler. The surface layers 6, 7 may further contain another component described above if necessary as long as the effect of the present invention is not impaired.

<Olefin-Based Resin>

Examples of the olefin-based resin include a polyethylene-based resin and a polypropylene-based resin. In light of improving stretchability of the surface layers 6, 7, the olefin-based resin is preferably polyethylene-based resin. In light of improving heat resistance of the surface layers 6, 7, the olefin-based resin is preferably polypropylene-based resin.

Examples of the polyethylene-based resin include low-density polyethylene (LDPE), linear low-density polyethylene (LLDPE), etc. In light of reducing tackiness of the elastomer component, the polyethylene-based resin is preferably low-density polyethylene (density: 0.910 $g/cm^3$ to 0.930 $g/cm^3$), and in light of improving stretchability, the polyethylene-based resin is preferably linear low-density polyethylene (density: 0.910 $g/cm^3$ to 0.920 $g/cm^3$).

Examples of the polypropylene-based resin include homo polypropylene (H-PP) obtained by polymerizing propylene alone, random polypropylene (R-PP) obtained by copolymerizing propylene and ethylene, block polypropylene (block polypropylene obtained by copolymerizing with ethylene, B-PP) obtained by polymerizing homo polypropylene and then copolymerizing ethylene and propylene in the presence of homo polypropylene, propylene-based elastomer such as propylene-ethylene copolymer, etc. Since ethylene copolymerization decreases stereoregularity and then crystallinity, in light of improving flexibility and heat resistance as compared with polyethylene resin, the polypropylene-based resin is preferably random polypropylene (ethylene unit content rate: 5 mass% or less, melting point: 135°C to 150°C) among these, and in light of improving stretchability, the polypropylene-based resin is preferably propylene-based elastomer (propylene unit content rate: 70 mass% to 95 mass%, ethylene unit content rate: 5 mass% to 30 mass%) among these.

One kind of these olefin-based resin may be used alone, or two or more kinds of them may be used in combination. For example, in light of improving stretchability, the olefin-based resin may be a blend of random polypropylene and linear low-density polyethylene. In light of suppressing blocking in the elastomer component, the olefin-based resin may be a blend of propylene-based elastomer and low-density polyethylene.

The content of the olefin-based resin in the entire surface layer 6 (or the surface layer 7) (i.e., the entire olefin-based resin composition) is preferably from 30 mass% to 85 mass% inclusive relative to 100 mass% of the entire surface layer. This is because, when the content of the olefin-based resin is less than 30 mass%, the content of the inorganic filler in the surface layer increases, and thus the stretchability may decrease, and when the content of the olefin-based resin is more than 85 mass%, the content of the inorganic filler in the surface layer decreases, and thus blocking may occur.

<Inorganic Filler>

The inorganic filler is a component for imparting slipperiness to the surfaces of the surface layers 6, 7 to further suppress blocking in the stretchable film 1.

Examples of the inorganic filler include calcium carbonate, zeolite, silica, titanium oxide, calcium oxide, magnesium oxide, zinc oxide, clay, mica, barium sulfate, magnesium hydroxide, etc. One kind of these inorganic fillers may be used alone, or two or more kinds of them may be used in combination.

The content of the inorganic filler in the entire surface layer 6 (or the surface layer 7) (i.e., the entire olefin-based resin composition) is preferably from 15 mass% to 70 mass% inclusive relative to 100 mass% of the entire surface layer. This is because, when the content of the inorganic filler is less than 15 mass%, the content of the inorganic filler in the surface layer decreases, and thus blocking may occur, and when the content of the inorganic filler is more than 70 mass%, the content of the inorganic filler in the surface layer increases, and thus the stretchability may decrease.

The inorganic filler has a mean particle diameter of preferably 0.8 $\mu$m to 10 $\mu$m. When the inorganic filler has a mean particle diameter of 0.8 $\mu$m or more, secondary aggregation or the like of the inorganic filler is suppressed, dispersibility in the resin becomes excellent, and slipperiness is improved. When the inorganic filler has a mean particle diameter of 10 $\mu$m or less, the texture is improved.

<Method for Producing Stretchable Film>

Next, a method for producing the stretchable film according to the present embodiment will be described in detail below.

The stretchable film according to the present embodiment is produced by molding a raw material containing the styrene-based elastomer, the olefin-based resin, and the inorganic filler described above into a film shape using an extruder.

More specifically, first, the styrene-based elastomer and if necessary, the other components described above are mixed at a predetermined blend ratio to obtain a resin mixture for forming an elastomer layer. Also, the olefin-based resin, the inorganic filler, and if necessary, the other components are mixed at a predetermined blend ratio, and the resultant is then extruded in the form of a strand by, for example, a co-rotation twin-screw extruder equipped with a strand die and cut to obtain pellets for forming a surface layer.

Next, the resin mixture for forming an elastomer layer and the pellets for forming a surface layer are extrusion-molded at a predetermined temperature using an extruder equipped with a T-die to obtain a raw film before stretching, including an elastomer layer, a first surface layer (e.g., the surface layer 6 in FIG. 1) provided on the first surface of the elastomer layer, and a second surface layer (e.g., the surface layer 7 in FIG. 1) provided on the second surface of the elastomer layer, by cast film process method.

The raw film is subjected to uniaxial stretching to stretch the raw film, thereby producing a stretchable film 1 shown in FIGS. 1 and 2. The stretching method is not particularly limited, and examples thereof include gear stretching, roll stretching, tenter stretching, etc.

For example, a gear stretching can be performed using a pair of shaping rolls: a first shaping roll having, on the circumferential surface of its cylindrical roll body, multiple ridges extending in the circumferential direction; and a second shaping roll having, on the circumferential surface of its cylindrical roll body, multiple ridges extending in the circumferential direction, which are disposed to face each other with a predetermined clearance therebetween such that the ridges in the first shaping roll and grooves between the ridges in the second shaping roll are engaged with each other, and that grooves between the ridges in the first shaping roll and the ridges in the second shaping roll are engaged with each other.

Then, the raw film before gear stretching is passed through between the first shaping roll and the second shaping roll while rotating the first shaping roll and the second shaping roll, thereby forming stretched regions and non-stretched regions between ridges in the first shaping roll and the ridges in the second shaping roll.

In the gear stretching, the stretch ratio can be adjusted by adjusting the width W of the top portion of each ridge in the shaping roll, the height H of each ridge, the interval P between the top portions of the adjacent ridges, the engagement depth D between each ridge of the first shaping roll and each ridge of the second shaping roll, and the like. The stretch ratio in the gear stretching can be easily calculated from the principle of stretching by the Pythagorean theorem.

The uniaxial stretching described above is stretching in one of the MD or the TD shown in FIG. 2. Biaxial stretching of the stretching in both the MD and the TD may also be performed.

The stretching temperature in the uniaxial stretching is usually room temperature ($23°C \pm 2°C$). This is because the stretching at room temperature can remove residual strain remaining in film molding, and has an effect of reducing permanent strain. In addition, in the case of stretching at a temperature higher than room temperature, the orientation proceeds in the stretching direction, and thus the permanent strain may increase.

The stretch ratio in the uniaxial stretching is from 1.5 to 9 inclusive. This is because when the stretch ratio is less than 1.5, residual strain remaining in film molding may not be removed, and when the stretch ratio is larger than 9, the film may break when stretched. The term "stretch ratio" used herein refers to multiples of the length of the film after stretching in the stretching direction with respect to the length of the film before the stretching. In the gear stretching, the stretch ratio refers to the multiple of the length of the film in the stretched region relative to the length of the film before stretching. For example, if an interval between the top portion of the ridge in the first shaping roll and the top portion of the ridge in the second shaping roll, adjacent to each other (the width of a portion to be stretched of the film before gear stretching) is 1 mm, and the engagement depth is $\sqrt{3}$ mm, the width of the stretched portion of the film becomes 2 mm: the stretch ratio is two.

Since the stretchable film of this embodiment produced by the method described above has a hysteresis loss of less than 36%, in the stretchable film 1 including an elastomer layer 5 and surface layers 6, 7 stacked on the surfaces of the elastomer layer 5, it becomes possible to suppress the decrease in stretchability of the surface layers 6, 7, resulting in the stretchable film 1 having excellent stretchability. The hysteresis loss is preferably less than 30%, more preferably less than 25%.

The term "hysteresis loss" used herein refers to one calculated by the following method.

A strip-shaped test piece is cut from the stretchable film. The test piece has 100 mm in one direction of the film and 25 mm in a direction orthogonal to the one direction. The test piece is then fixed to grippers of a tester (e.g., Autograph AG-5000A manufactured by SHIMADZU CORPORATION) so that the distance between the grippers becomes 25 mm. The test piece is then elongated in its longitudinal direction at a speed 254 mm/min so that the strain (elongation) calculated by the following equation (1) is 100%. The integral area in the stress-strain curve (S-S curve) during this elongation is defined as S1, and the integral area in the stress-strain curve (S-S curve) during contraction of the test piece at the same speed as for the elongation, immediately after the elongation so that the load (N/25 mm) of the test piece reaches 0 is defined as S2. Then, the hysteresis loss [%] is calculated from the following equation (2).

$$\text{Strain [\%]} = (L1 - L0)/L0 \times 100 \quad (1)$$

$$\text{Hysteresis Loss [\%]} = S/S1 \times 100 \quad (2)$$

L0 is a distance (mm) between the grippers before the elongation, and L1 is a distance (mm) between the grippers after the elongation. S1 is the integral area of the stress-strain curve during the elongation from 0% to 100% strain, S2 is the integral area of the stress-strain curve during the contraction from 100% strain to 0 stress, and S is an integral area (i.e., S = S1 - S2) of a stress-strain curve, obtained by subtracting the integral area S1 from the integral area S2.

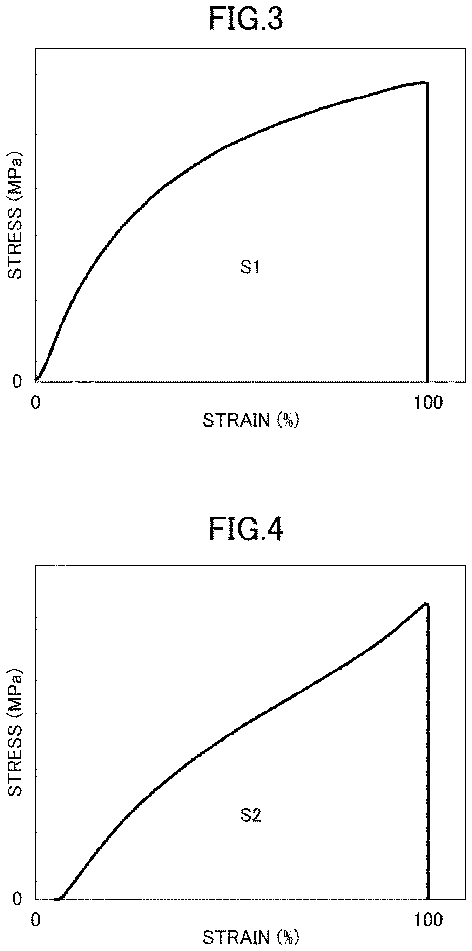

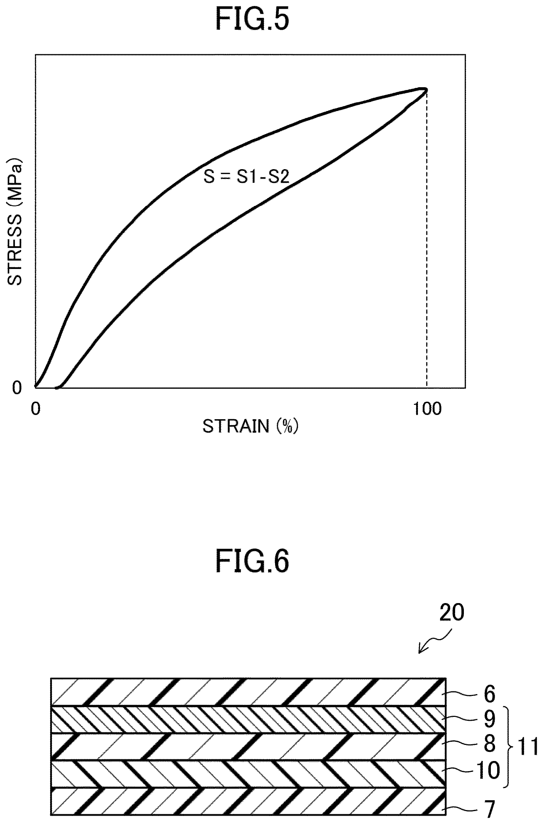

That is, the hysteresis loss can be determined by the above equation (2), by subtracting the integral area S2 of the stress-strain curve during the contraction from 100% strain to 0 stress, shown in FIG. 4, from the integral area S1 of the stress-strain curve during the elongation from 0% to 100% strain, shown in FIG. 3, to obtain the integral area (i.e., the integral area S shown in FIG. 5) of the stress-strain curve, and dividing the integral area by the integral area S1.

In addition, when the stretchable film of this embodiment has a permanent strain in the stretching direction of 7% or less, the degree of plastic deformation is small, resulting in excellent stretchability. The permanent strain is preferably 6.5% or less.

The term "permanent strain" used herein refers to a strain calculated by the following method.

A strip-shaped test piece is cut from the stretchable film. The test piece has 100 mm in one direction of the film and 25 mm in a direction orthogonal to the one direction. The test piece is then fixed to grippers of a tester (e.g., Autograph AG-5000A manufactured by SHIMADZU CORPORATION) so that the distance between the grippers becomes 25 mm. The test piece is then elongated in its longitudinal direction at a speed 254 mm/min so that the strain (elongation) calculated by the following equation (1) reaches 100%. Immediately after the elongation, the test piece is contracted at the same speed. The permanent strain [%] is then calculated from the following equation (3).

$$\text{Strain } [\%] = (L1 - L0)/L0 \times 100 \ (1)$$

$$\text{Permanent Strain } [\%] = (L2 - L0)/L0 \times 100 \ (3)$$

L0 is a distance (mm) between the grippers before the elongation, L1 is a distance (mm) between the grippers after the elongation, and L2 is a distance (mm) between the grippers when a load (N/25 mm) of the test piece becomes 0 during the contraction.

In light of transportability of the stretchable film, the stress at the time of stretching in the MD such that the strain (elongation) calculated by the above equation (1) becomes 10% (i.e., the stress at 10% elongation) is preferably 1.2 $N/mm^2$ or more, more preferably 2 $N/mm^2$ or more, yet more preferably 3 $N/mm^2$ or more.

The strain at the time of stretching in the TD such that the strain (elongation) calculated by the above equation (1) becomes 50% (i.e., stress at 50% elongation) is preferably 0.5 $N/mm^2$ to 1.5 $N/mm^2$, more preferably 0.8 $N/mm^2$ to 1.2 $N/mm^2$. When the stress at 50% elongation is less than 0.5 $N/mm^2$, the stress is too low, making it necessary to increase the film thickness in order to achieve appropriate stretchability, which can lead to increased costs. In addition, when the stress at 50% elongation exceeds 1.5 $N/mm^2$, the stress is too high, making it necessary to reduce the film thickness; however, if the film becomes too thin, handling may become difficult.

The thickness of the raw film is preferably 20 $\mu$m to 80 $\mu$m, more preferably 20 $\mu$m to 60 $\mu$m, yet more preferably 30 $\mu$m to 50 $\mu$m. When the thickness of the raw film is 20 $\mu$m or more, excellent stretchability is obtained. When the thickness of the raw film is 80 $\mu$m or less, the stretchable film can be made thin, thereby lowering rigidity and obtaining excellent stretchability.

The thickness of the elastomer layer 5 in the raw film is preferably 20 $\mu$m to 40 $\mu$m, more preferably 25 $\mu$m to 35 $\mu$m, yet more preferably 30 $\mu$m to 33 $\mu$m. When the thickness of the elastomer layer 5 is 20 $\mu$m or more, the stretchable film 1 can obtain sufficient stretchability, and can be stably wound around a roll during film molding. When the thickness of the elastomer layer 5 is 40 $\mu$m or less, the stretchable film 1 can be made thin, and the stress of the stretchable film can be reduced.

The thickness of the surface layer 6, 7 in the raw film is preferably 1 $\mu$m to 10 $\mu$m, more preferably 1 $\mu$m to 5 $\mu$m, yet more preferably 1 $\mu$m to 3 $\mu$m, most preferably 1.2 $\mu$m to 2.2 $\mu$m. When the thickness of the surface layer 6, 7 is 1 $\mu$m or more, blocking of the stretchable film 1 after stretching is sufficiently suppressed. When the thickness of the surface layer 6, 7 is 10 $\mu$m or less, sufficient stretchability of the stretchable film 1 can be obtained. The surface layers 6, 7 may have the same thickness or different thicknesses.

In particular, for the stretchable film 1 having a small ratio of the thickness of the surface layer 6, 7 to the thickness of the entire stretchable film, the ratio between the thickness of the surface layer 7 (or the surface layer 8) and the thickness of the elastomer layer 5 in the raw film is preferably the surface layer : the elastomer layer = 1:5 to 1:35, more preferably 1:8 to 1:30, yet more preferably 1:15 to 1:27 in light of improving stretchability.

When the stretchable film 1 is stretched at room temperature, the thickness of the stretchable film 1 after the stretching is 85% to 95% of the thickness of the raw film. For the gear stretching, an unstretched portion has the same thickness as that of the raw film, and a stretched portion is 85% to 95% of the raw film.

According to the above method, in the present embodiment, the stretchable film 1 in which the surface layers 6, 7 are stacked on the elastomer layer 5 can have excellent stretchability.

When the stretchable film is multilayered, each layer may have the same or different composition and thickness. When the stretchable film is multilayered, the thickness thereof means the thickness of the entire multilayer.

[Second Embodiment]

Next, the second embodiment of the present invention will be described. The same components as those of the first embodiment are denoted by the same reference numerals, and description thereof is omitted.

FIG. 6 is a sectional view of a stretchable film according to the present embodiment. As illustrated in FIG. 6, the stretchable film 20 of this embodiment includes an elastomer layer 11, and surface layers 6, 7 stacked on the surfaces of the elastomer layer 11.

(Elastomer Layer)

The elastomer layer 11 is a layer which imparts stretchability to the stretchable film 1. As illustrated in FIG. 6, the elastomer layer 11 has a three-layer structure including a core layer 8 and intermediate layers 9, 10 stacked on the surfaces of the core layer 8, and the intermediate layer 9 is provided between the core layer 8 and the surface layer 6, and the intermediate layer 10 is provided between the core layer 8 and the surface layer 7.

<Core Layer>

The core layer 8 contains an olefin-based elastomer, which is a thermoplastic elastomer. By using the olefin-based elastomer, it becomes possible to improve the stress at 50% elongation in the TD, as compared with the case where only the elastomer layer 5 containing a styrene-based elastomer is provided, as in the above-described first embodiment.

The olefin-based elastomer used in the present invention can be, for example, a copolymer containing, as a main component, an olefin having 3 or more carbon atoms.

More specifically, examples of the olefin-based elastomer include $\alpha$-olefin copolymers such as a propylene-ethylene copolymer, a propylene-ethylene-1-butene copolymer, 1-butene-ethylene copolymer, a 1-butene-propylene copolymer, a 4-methylpentene-1-propylene copolymer, 4-methylpentene-1-1-butene copolymer, 4-methylpentene-1-propylene-1-butene copolymer, and a propylene-1-butene copolymer. Alternatively, the olefin-based elastomer used can be an elastomer in which the above-described elastomer is dispersed in a matrix of crystalline polyolefin. Among them, the olefin-based elastomer is preferably a propylene-based elastomer (olefin-based elastomer in which the hard segments are made of polypropylene) such as a propylene-ethylene copolymer, because of its stretchability. One kind of these olefin-based elastomers may be used alone, or two or more kinds of them may be used in combination.

Examples of the soft segments of the olefin-based elastomer include EPDM, EPM, EBM, IIR, hydrogenated styrene-butadiene rubber (HSBR), NBR, and acrylic rubber (ACM).

For the propylene-based elastomer, the propylene unit content rate relative to total units of the propylene-based elastomer is preferably 70 mass% to 95 mass%, more preferably 80 mass% to 90 mass%. For example, in the case of using the propylene-ethylene copolymer, the propylene unit content rate relative to the total units is preferably 70 mass% to 95 mass% (i.e., the ethylene unit content rate is preferably 5 mass% to 30 mass%). When the content rate of the propylene unit, which are hard segments, is 70 mass% or higher, the strength is improved, thereby obtaining excellent moldability, and when the content rate is 95 mass% or lower, excellent stretchability due to elasticity of the soft segments is obtained.

The MFR of the propylene-based elastomer is preferably 0.5 g/10 min to 10 g/10 min, more preferably 2.0 g/10 min to 8.0 g/10 min. When the MFR of the propylene-based elastomer is larger than 10 g/10 min, the melt viscosity may become too low, making it difficult to control the thickness during film molding. When the MFR of the propylene-based elastomer is less than 0.5 g/10 min, the melt fracture may occur during film molding, which may result in a rough film surface. When the MFR of the propylene-based elastomer is less than 2.0 g/10 min, the film may break during high-speed extrusion molding in the film molding because of the high melt viscosity.

The density of the propylene-based elastomer is preferably less than 0.900 g/cm$^3$. When the density of the propylene-based elastomer is 0.900 g/cm$^3$ or more, stretchability of the film may decrease.

The core layer 8 may contain another component besides the propylene-based elastomer described above as long as the effect of the present invention is not impaired. Examples of the other components include other resins than the propylene-based elastomer such as an olefin-based resin, an amide-based anti-blocking agent (such as octadecanamide), a plasticizer, an ultraviolet absorber, an antioxidant, a weathering agent, an antistatic agent, a colorant, an anti-fogging agent, metal soap, wax, an antifungal agent, an antibacterial agent, a nucleating agent, a flame retardant, a lubricant, etc. These other components may be added to the material for the stretchable film in the form of masterbatch.

<Intermediate Layer>

As the intermediate layers 9, 10, the same elastomer layer as the elastomer layer 5 (i.e., the elastomer layer containing the styrene-based elastomer) described in the first embodiment can be used.

When the SIS elastomer is used in combination with other SIS elastomers (for example, SIS elastomers having different styrene unit content rates are blended), the styrene unit content rate relative to the total units of the SIS elastomer is preferably from 9 mass% to 35 mass% inclusive. When an SIS elastomer having a high styrene unit content rate (i.e., the styrene unit content rate exceeds 30 mass%) and an SIS elastomer having a low styrene unit content rate (i.e., the styrene unit content rate is 30 mass% or less) are blended, in light of suppressing the decrease in stretchability in the TD while improving the stress in the MD, the blend ratio between the SIS elastomer having a high styrene unit content rate and the SIS elastomer having a low styrene unit content rate is preferably the SIS elastomer having high styrene unit content rate: the SIS elastomer having low styrene unit content rate = 20:80 to 50:50. When the blend ratio is within the range, the Shore A hardness can be set to 30 or more in order to improve the stress at 10% elongation [N/mm$^2$] in the MD, and the styrene unit content rate can be set to be 35 mass% or less relative to the entire elastomer layer in order to enhance stretchability in the TD, sacrificed due to the Shore A hardness.

<Method for Producing Stretchable Film>

Next, a method for producing the stretchable film according to the present embodiment will be described in detail below.

The stretchable film of the present invention is produced by molding a raw material containing an olefin-based elastomer, a styrene-based elastomer, an olefin-based resin, and an inorganic filler, which are described above, into a film by using an extruder.

More specifically, first, the olefin-based elastomer and if necessary, the other components described above are mixed at a predetermined blend ratio to obtain a resin mixture for forming a core layer. Further, in the same manner, the styrene-based elastomer and if necessary, the other components described above are mixed at a predetermined blend ratio to obtain a resin mixture for forming an intermediate layer. Also, the olefin-based resin, the inorganic filler, and if necessary, the other components are mixed at a predetermined blend ratio, and the resultant is then extruded in the form of a strand by, for example, a co-rotation twin-screw extruder equipped with a strand die and cut to obtain pellets for forming a surface layer.

Next, the resin mixture for forming a core layer, the resin mixture for forming an intermediate layer, and the pellets for forming a surface layer are extrusion-molded at a predetermined temperature using an extruder equipped with a T-die to obtain, by a cast film process method, a raw film before stretching, including: a core layer; an elastomer layer (i.e., the elastomer layer 11 in FIG. 6) that includes a first intermediate layer (i.e., the intermediate layer 9 in FIG. 6) provided on the first surface of the core layer and a second intermediate layer (i.e., the intermediate layer 10 in FIG. 6) provided on the second surface of the core layer; a first surface layer (i.e., the surface layer 6 in FIG. 6) provided on the first surface of the elastomer layer; and a second surface layer (i.e., the surface layer 7 in FIG. 6) provided on the second surface of the elastomer layer.

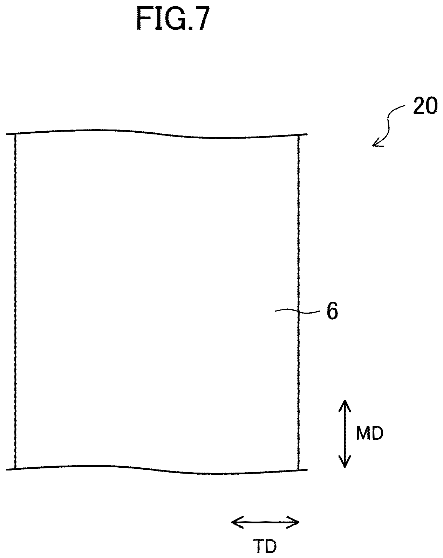

The raw film is subjected to uniaxial stretching in either one of the MD or TD shown in FIG. 7 to stretch the raw film, thereby obtaining the stretchable film 20 shown in FIGS. 6 and 7. The stretching method is not particularly limited as in the first embodiment, and examples thereof include gear stretching, roll stretching, tenter stretching, etc. Biaxial stretching of the stretching in both the MD and the TD may also be performed.

As in the first embodiment, the stretching temperature in the uniaxial stretching is generally room temperature (23°C ± 2°C), and the stretch ratio in the uniaxial stretching is from 1.5 to 9 inclusive.

Since the stretchable film of this embodiment produced by the method described above has a hysteresis loss of less than 36% as in the first embodiment, in the stretchable film 20 including an elastomer layer 11 and surface layers 6, 7 stacked on the surfaces of the elastomer layer 11, it becomes possible to suppress the decrease in stretchability of the surface layers 6, 7, resulting in the stretchable film 20 having excellent stretchability. The hysteresis loss is preferably less than 30%, more preferably less than 25%.

Since the stretchable film of this embodiment has a permanent strain of 7% or less in the stretching direction as in the first embodiment, excellent stretchability can be obtained. The permanent strain is preferably 6.5% or less.

As in the first embodiment, in light of transportability of the stretchable film, the stress at the time of stretching in the MD such that the strain (elongation) calculated by the above equation (1) becomes 10% (i.e., stress at 10% elongation) is preferably 1.2 N/mm$^2$ or more, more preferably 2 N/mm$^2$ or more, yet more preferably 3 N/mm$^2$ or more.

As in the first embodiment, in light of suppressing an increase in cost, the stress at the time of stretching in the TD such that the strain (elongation) calculated by the above equation (1) becomes 50% (i.e., stress at 50% elongation) is preferably 0.5 N/mm$^2$ to 1.5 N/mm$^2$, more preferably 0.8 N/mm$^2$ to 1.2 N/mm$^2$.

As in the first embodiment, the raw film has a thickness of preferably 20 μm to 80 μm, more preferably 20 μm to 60

μm, yet more preferably 30 μm to 50 μm. The thickness of the elastomer layer 11 in the raw film is preferably 20 μm to 40 μm, more preferably 25 μm to 35 μm, yet more preferably 30 μm to 33 μm.

The thickness of the core layer 8 in the raw film is preferably 5 μm to 20 μm, more preferably 7 μm to 18 μm, yet more preferably 9 μm to 17 μm. The total thickness of the intermediate layers 9, 10 in the raw film is preferably 10 μm to 25 μm, more preferably 12 μm to 22 μm, yet more preferably 14 μm to 20 μm.

In particular, in the stretchable film 20 in which the elastomer layer 11 has a three-layer structure including a core layer 8 and intermediate layers 9, 10, in light of suppressing the decrease in stretchability in the TD while improving the stress at 50% elongation in the TD, the ratio between the thickness of the core layer 8 and the total thickness of the intermediate layers 9, 10 in the raw film is preferably the thickness of core layer : the total thickness of intermediate layers = 20:80 to 50:50. That is, since the improvement in the stress at 50% elongation in the TD and the improvement in stretchability in the TD are in a trade-off relationship, if the ratio between the thickness of the core layer and the total thickness of the intermediate layers is within the above range, the improvement in stress at 50% elongation in the TD and the improvement in stretchability in the TD can be both achieved.

As in the first embodiment, the thickness of the surface layer 6, 7 in the raw film is preferably 1 μm to 10 μm, more preferably 1 μm to 5 μm, yet more preferably 1 μm to 3 μm, most preferably 1.2 μm to 2.2 μm.

As in the first embodiment, the ratio between the thickness of the surface layer 7 (or the surface layer 8) and the thickness of the elastomer layer 11 in the raw film is preferably the surface layer : the elastomer layer = 1:5 to 1:35, more preferably 1:8 to 1:30, yet more preferably 1:15 to 1:27.

As in the first embodiment, when the stretchable film 20 is stretched at room temperature, the thickness of the stretchable film 20 after the stretching is 85% to 95% of the thickness of the raw film. For the gear stretching, an unstretched portion has the same thickness as that of the raw film, and a stretched portion is 85% to 95% of the raw film.

According to the above method, in the present embodiment, the stretchable film 20 in which the surface layers 6, 7 are stacked on the elastomer layer 11 can have excellent stretchability.

When the stretchable film is multilayered, each layer may have the same or different composition and thickness. When the stretchable film is multilayered, the thickness thereof means the thickness of the entire multilayer.

[Examples]

The present invention will be described below based on Examples. Note that the present invention shall not be limited to these examples. These examples may be modified and changed based on the intent of the present invention. Such a change and modification shall not be excluded from the scope of the present invention.

Materials used for production of a stretchable film are shown below:

(1) PP1: random polypropylene (manufactured by Prime Polymer Co., Ltd., trade name: F227, density: 0.90 g/cm$^3$, MFR: 6.7 g/10 min (230°C))

(2) PP2: random polypropylene (manufactured by Prime Polymer Co., Ltd., trade name: F327, density: 0.90 g/cm$^3$, MFR: 7.0 g/10 min (230°C))

(3) PP3: random polypropylene (manufactured by Prime Polymer Co., Ltd., trade name: F-744NP, density: 0.90 g/cm$^3$, MFR: 7.0 g/10 min (230°C))

(4) PP4: random polypropylene (manufactured by Prime Polymer Co., Ltd., trade name: 730NV, density: 0.90 g/cm$^3$, MFR: 6.2 g/10 min (230°C))

(5) PP5: random polypropylene (manufactured by Sumitomo Chemical Co., Ltd., trade name: FLX86E1, density: 0.89 g/cm$^3$, MFR: 7.9 g/10 min (230°C))

(6) PP6: random polypropylene (manufactured by SunAllomer Ltd., trade name: PF621s, density: 0.90 g/cm$^3$, MFR: 6.5 g/10 min (230°C))

(7) PP7: random polypropylene (manufactured by SunAllomer Ltd., trade name: PC630s, density: 0.90 g/cm$^3$, MFR: 7.5 g/10 min (230°C))

(8) PP8: random polypropylene (manufactured by SunAllomer Ltd., trade name: PC630a, density: 0.90 g/cm$^3$, MFR: 7.5 g/10 min (230°C))

(9) Inorganic filler: calcium carbonate, mean particle diameter: 1.8 μm (manufactured by SHIRAISHI CALCIUM KAISHA, LTD., trade name: PO-120B-10)

(10) SIS elastomer 1 (manufactured by ZEON CORPORATION, trade name: Quintac3390, styrene unit content rate: 48 mass%, density: 0.98 g/cm$^3$, Shore A hardness: 65, MFR: 14 g/10 min (200°C))

(11) SIS elastomer 2 (manufactured by ZEON CORPORATION, trade name: Quintac3290, styrene unit content rate: 35 mass%, density: 0.93 g/cm$^3$, Shore A hardness: 55, MFR: 10 g/10 min (200°C))

(12) SIS elastomer 3 (manufactured by ZEON CORPORATION, trade name: Quintac3440, styrene unit content rate: 18 mass%, density: 0.93 g/cm$^3$, Shore A hardness: 45, MFR: 10 g/10 min (200°C))

(13) SIS elastomer 4 (manufactured by ZEON CORPORATION, trade name: Quintac3620, styrene unit content rate:

14 mass%, density: 0.93 g/cm$^3$, Shore A hardness: 36, MFR: 9 g/10 min (200°C))

(14) SIS elastomer 5 (manufactured by ZEON CORPORATION, trade name: Quintac3433N, styrene unit content rate: 16 mass%, density: 0.93 g/cm$^3$, Shore A hardness: 21, MFR: 12 g/10 min (200°C))

(15) SIS elastomer 6 (manufactured by KANEKA CORPORATION, trade name: SIBSTAR 073T, styrene unit content rate: 30 mass%, density: 0.954 g/cm$^3$, Shore A hardness: 45, MFR: 6 g/10 min (230°C))

(16) SIS elastomer 7 (manufactured by Kraton Corporation, trade name: KRATON D1161, styrene unit content rate: 15 mass%, density: 0.92 g/cm$^3$, Shore A hardness: 32, MFR: 12 g/10 min (200°C))

(17) SIS elastomer 8 (manufactured by Kraton Corporation, trade name: KRATON D1126, styrene unit content rate: 21 mass%, density: 0.92 g/cm$^3$, Shore A hardness: 39, MFR: 22 g/10 min (200°C))

(18) Propylene-based elastomer (manufactured by Exxon Mobil Corporation, trade name: Vistamaxx (registered trademark) 6102FL, polyethylene content rate: 16%, density: 0.862 g/cm$^3$, Shore A hardness: 67, MFR: 3.0 g/10 min (230°C))

(19) Ethylene-based elastomer (manufactured by Dow Chemical Company, trade name: Infuse9007, ethylene-octene copolymer, density: 0.864/cm$^3$, Shore A hardness: 64, MFR: 0.5 g/10 min (190°C))

(20) SBS elastomer (manufactured by Asahi Kasei Chemicals Corporation, trade name: Tufprene A, styrene unit content rate: 40 mass%, density: 0.95 g/cm$^3$, Shore A hardness: 85, MFR: 2.6 g/10 min (190°C))

(21) SEBS elastomer (manufactured by Asahi Kasei Chemicals Corporation, trade name: Tuftec H1221, styrene unit content rate: 12 mass%, density: 0.89 g/cm$^3$, Shore A hardness: 42, MFR: 4.5 g/10 min (230°C))

(Example 1)

<Production of Stretchable Film>

First, materials shown in Table 1 were mixed, thereby preparing a resin mixture for forming an elastomer layer and a resin mixture for forming a surface layer of Example 1 each having the composition (parts by mass) shown in Table 1. Next, the resin mixture for forming a surface layer was extruded into a strand shape at 200°C using a co-rotation twin-screw extruder equipped with a strand die (manufactured by JSW, trade name: TEX28V-42CW-4V), and cut to obtain pellets for forming a surface layer.

Then, the resin mixture for forming an elastomer layer and the pellets for forming a surface layer were extrusion-molded at 200°C using an extruder equipped with a T-die (manufactured by Labotech Inc.) to obtain a film having an elastomer layer, a first surface layer provided on a first surface of the elastomer layer, and a second surface layer provided on a second surface of the elastomer layer, by a cast film process method. The film was then wound up by a winding roll, thereby obtaining a raw film before stretching, having a thickness shown in Table 1.

The raw film was subjected to Gear stretching (the stretch ratio: 5) in the TD at room temperature (23°C ± 2°C), thereby producing a stretchable film.

<Calculation of Styrene Unit Content Rate in Elastomer Layer (Relative to Total Units of SIS Elastomer)>

The styrene unit content rate [%] of the elastomer layer in the produced stretchable film was calculated. More specifically, in Example 1, the stretchable film contains 100 parts by mass of the SIS elastomer 3 (the styrene unit content rate: 18 mass%) relative to 100 parts by mass of the elastomer layer. Thus, the styrene unit content rate in the elastomer layer is $18 \times (100/100) = 18$ mass%. Table 1 shows the results.

<Measurement of Shore A Hardness of Elastomer Layer>

The Shore A hardness of the elastomer layer was measured at a temperature of 23°C in accordance with JIS K 6253 by using a type A durometer (spring type durometer). Table 1 shows the results.

<Calculation of Content Rate of Styrene-Based Elastomer in Entire Stretchable Film>

The content rate [%] of the styrene-based elastomer in the entire stretchable film produced was calculated. More specifically, in Example 1, the stretchable film contains 100 parts by mass of the SIS elastomer 3 (the styrene-based elastomer) relative to 100 parts by mass of the elastomer layer, and the thickness of the elastomer layer is 28 $\mu$m with respect to the thickness (35 $\mu$m) of the entire stretchable film. Thus, the content rate of the styrene-based elastomer in the entire stretchable film is $(28/35) \times 100 = 80$ mass%. Table 1 shows the results.

<Measurement of Hysteresis Loss>

A strip-shaped test piece was cut from the stretchable film produced. The test piece had 100 mm in one direction (TD) of the film and 25 mm in the direction (MD) orthogonal to the one direction. The test piece was then fixed to grippers of a precision universal tester (Autograph AG-5000A manufactured by SHIMADZU CORPORATION) so that the distance between the grippers was 25 mm. The test piece was then elongated in its longitudinal direction at a speed 254 mm/min so that the strain (elongation) calculated by the above equation (1) reached 100%. The integral area in the stress-strain curve (S-S curve) during this elongation was defined as S1, and the integral area in the stress-strain curve (S-S curve) during contraction of the test piece at the same speed as for the elongation, immediately after the elongation so that the load (N/25 mm) of the test piece reached 0 was defined as S2. Then, the hysteresis loss [%] was calculated from the above equation (2). Table 1 shows the results.

<Measurement of Permanent Strain>

A strip-shaped test piece was cut from the stretchable film produced. The test piece had 100 mm in one direction (TD) of the film and 25 mm in the direction (MD) orthogonal to the one direction. The test piece was then fixed to grippers of a precision universal tester (Autograph AG-5000A manufactured by SHIMADZU CORPORATION) so that the distance between the grippers was 25 mm. The test piece was then elongated in its longitudinal direction at a speed 254 mm/min so that the strain (elongation) calculated by the above equation (1) reached 100%. Immediately after the elongation, the test piece was contracted at the same speed. The permanent strain [%] in the TD was then calculated from the above equation (3). The test was performed at room temperature (23°C $\pm$ 2°C). Table 1 shows the results.

<Measurement of Stress at 10% Elongation>

A strip-shaped test piece was cut from the stretchable film produced. The test piece had 100 mm in one direction (MD) of the film and 25 mm in the direction (TD) orthogonal to the one direction. The test piece was then fixed to grippers of a precision universal tester (Autograph AG-5000A manufactured by SHIMADZU CORPORATION) so that the distance between the grippers was 25 mm. The test piece was then elongated in its longitudinal direction at a speed 254 mm/min so that the strain (elongation) calculated by the above equation (1) reached 10%. The stress [N/mm$^2$] at 10% elongation in the MD in this elongation was calculated. Table 1 shows the results.

<Measurement of Stress at 50% Elongation>

A strip-shaped test piece was cut from the stretchable film produced. The test piece had 100 mm in the direction (TD) orthogonal to one direction of the film and 25 mm in the one direction (MD). The test piece was then fixed to grippers of a precision universal tester (Autograph AG-5000A manufactured by SHIMADZU CORPORATION) so that the distance between the grippers was 25 mm. The test piece was then elongated in a direction orthogonal to the longitudinal direction at a speed 254 mm/min so that the strain (elongation) calculated by the above equation (1) reached 50%, and the stress [N/mm$^2$] at 50% elongation in the TD in this elongation was calculated. Table 1 shows the results.

(Examples 2 to 9)

Each raw film having a thickness shown in Table 1 was stretched in the same manner as in Example 1 except that the composition (parts by mass) of the surface layer, the composition (parts by mass) of the elastomer layer, and the ratio between the thickness of the surface layer and the thickness of the elastomer layer were changed to the conditions shown in Table 1. Thus, each stretchable film was produced.
Then, in the same manner as in Example 1, calculation of the styrene unit content rate in the elastomer layer, measurement of the Shore A hardness of the elastomer layer, calculation of the content rate of the styrene-based elastomer in the entire stretchable film, measurement of hysteresis loss, measurement of permanent strain, measurement of stress at 10% elongation, and stress at 50% elongation were conducted. Table 1 shows the results.

(Example 10)

<Production of Stretchable Film>

First, materials shown in Table 2 were mixed, thereby preparing a resin mixture for forming an elastomer layer (core layer), a resin mixture for forming an elastomer layer (intermediate layer), and a resin mixture for forming a surface layer, of Example 10, each having composition (parts by mass) shown in Table 2. Next, the resin mixture for forming a surface layer was extruded into a strand shape at 200°C using a co-rotation twin-screw extruder equipped with a strand die (manufactured by JSW, trade name: TEX28V-42CW-4V), and cut to obtain pellets for forming a surface layer.

Next, the resin mixture for forming an elastomer layer (core layer), the resin mixture for forming an elastomer layer (intermediate layer), and the pellets for forming a surface layer were extrusion-molded at 200°C using an extruder (manufactured by Labotech Inc.) equipped with a T-die to obtain, by a cast film process method, a film including: a core layer; an elastomer layer that includes a first intermediate layer provided on the first surface of the core layer and a second intermediate layer provided on the second surface of the core layer; a first surface layer provided on the first surface of the elastomer layer; and a second surface layer provided on the second surface of the elastomer layer. The film was then wound up by a winding roll, thereby obtaining a raw film before stretching, having a thickness shown in Table 2.

The raw film was subjected to Gear stretching (the stretch ratio: 5) in the TD at room temperature (23°C ± 2°C), thereby producing a stretchable film.

Then, in the same manner as in Example 1, calculation of the styrene unit content rate in the elastomer layer, measurement of the Shore A hardness of the elastomer layer, calculation of the content rate of the styrene-based elastomer in the entire stretchable film, measurement of hysteresis loss, measurement of permanent strain, measurement of stress at 10% elongation, and stress at 50% elongation were conducted. Table 2 shows the results.

<Calculation of Content Rate of Olefin-Based Elastomer in Entire Stretchable Film>

The content rate [%] of the olefin-based elastomer in the entire stretchable film produced was calculated. More specifically, in Example 10, the stretchable film contains 100 parts by mass of the propylene-based elastomer (the olefin-based elastomer) relative to 100 parts by mass of the core layer, and the thickness of the core layer is 15.54 μm with respect to the thickness (35 μm) of the entire stretchable film. Thus, the content rate of the olefin-based elastomer in the entire stretchable film is (15.54/35) × 100 = 44.4 mass%. Table 2 shows the results.

(Examples 11 to 25)

Each raw film having a thickness shown in Tables 2 and 3 was stretched in the same manner as in Example 10 except that the composition (parts by mass) of the surface layer, the composition (parts by mass) of the elastomer layer (core layer), the composition (parts by mass) of the elastomer layer (intermediate layer), and the ratio between the thickness of the surface layer and the thickness of the elastomer layer were changed to the conditions shown in Tables 2 and 3. Thus, each stretchable film was produced.

Then, in the same manner as in Example 1, calculation of the styrene unit content rate in the elastomer layer, measurement of the Shore A hardness of the elastomer layer, calculation of the content rate of the styrene-based elastomer in the entire stretchable film, measurement of hysteresis loss, measurement of permanent strain, measurement of stress at 10% elongation, and stress at 50% elongation were conducted. The content rate of the olefin-based elastomer in the entire stretchable film was calculated in the same manner as in Example 10. Tables 2 and 3 show the results.

(Comparative Examples 1 to 13)

Each raw film having a thickness shown in Table 4 was stretched in the same manner as in Example 1 except that the composition (parts by mass) of the surface layer, the composition (parts by mass) of the elastomer layer, and the ratio between the thickness of the surface layer and the thickness of the elastomer layer were changed to the conditions shown in Table 4. Thus, each stretchable film was produced.

Then, in the same manner as in Example 1, calculation of the styrene unit content rate in the elastomer layer, measurement of the Shore A hardness of the elastomer layer, calculation of the content rate of the styrene-based elastomer in the entire stretchable film, measurement of hysteresis loss, measurement of permanent strain, measurement of stress at 10% elongation, and stress at 50% elongation were conducted. In Comparative Examples 1 to 3, the content rate of the olefin-based elastomer in the entire stretchable film was calculated in the same manner as in Example 10. Table 4 shows the results.

(Comparative Examples 14 to 25)

Each raw film having a thickness shown in Table 5 was stretched in the same manner as in Example 10 except that the composition (parts by mass) of the surface layer, the composition (parts by mass) of the elastomer layer (core layer), the composition (parts by mass) of the elastomer layer (intermediate layer), and the ratio between the thickness of the surface layer and the thickness of the elastomer layer were changed to the conditions shown in Table 5. Thus, each stretchable film was produced.

Then, in the same manner as in Example 1, calculation of the styrene unit content rate in the elastomer layer, measurement of the Shore A hardness of the elastomer layer, calculation of the content rate of the styrene-based elastomer in the entire stretchable film, measurement of hysteresis loss, measurement of permanent strain, measurement of stress at 10% elongation, and stress at 50% elongation were conducted. In Comparative Examples 20 to 25, the content rate of the olefin-based elastomer in the entire stretchable film was calculated in the same manner as in Example 10. Table 5 shows the results.

[Table 1]

| | | | Example 1 | Example 2 | Example 3 | Example 4 | Example 5 | Example 6 | Example 7 | Example 8 | Example 9 |
|---|---|---|---|---|---|---|---|---|---|---|---|
| Surface Layer | Blend Ratio (parts by mass) | PP1 | 50 | 50 | 50 | 50 | 50 | 50 | 50 | 50 | 50 |
| | | Inorganic Filler | 50 | 50 | 50 | 50 | 50 | 50 | 50 | 50 | 50 |
| Elastomer Layer | Blend Ratio (parts by mass) | SIS Elastomer 1 | - | - | - | - | 30 | 40 | - | 30 | 40 |
| | | SIS Elastomer 2 | - | - | 50 | 30 | - | - | 80 | - | - |
| | | SIS Elastomer 3 | 100 | - | 50 | 70 | 70 | 60 | 20 | 70 | 60 |
| | | SIS Elastomer 4 | - | 100 | - | - | - | - | - | - | - |
| Thickness [μm] of Raw Film | | First Surface Layer | 3.5 | 3.5 | 1.72 | 1.19 | 1.17 | 1.27 | 3.5 | 3.5 | 3.5 |
| | | Elastomer Layer | 28.0 | 28.0 | 31.6 | 32.6 | 32.7 | 32.44 | 28.0 | 28.0 | 28.0 |
| | | Second Surface Layer | 3.5 | 3.5 | 1.72 | 1.19 | 1.17 | 1.27 | 3.5 | 3.5 | 3.5 |
| | | Entire Thickness | 35.0 | 35.0 | 35.0 | 35.0 | 35.0 | 35.0 | 35.0 | 35.0 | 35.0 |
| | | Thickness of Surface Layer : Thickness of Elastomer Layer | 1 : 8 | 1 : 8 | 1 : 18.4 | 1 : 27.4 | 1 : 27.9 | 1 : 25.5 | 1 : 8 | 1 : 8 | 1 : 8 |
| Styrene Unit Content Rate [%] in Elastomer Layer | | | 18.0 | 14.0 | 26.5 | 23.1 | 27.0 | 30.0 | 31.6 | 27.0 | 30.0 |
| Shore A Hardness [-] of Elastomer Layer | | | 45.0 | 36.0 | 50.0 | 48.0 | 51.0 | 53.0 | 53.0 | 51.0 | 53.0 |
| Content Rate [%] of Styrene-Based Elastomer in Entire Stretchable Film | | | 80.0 | 80.0 | 90.2 | 93.2 | 93.3 | 92.7 | 80.0 | 80.0 | 80.0 |
| Evaluations | | Hysteresis Loss [%] | 27.2 | 32.6 | 29.1 | 24.6 | 24.8 | 28.9 | 33.7 | 32.3 | 33.4 |
| | | Permanent Strain [%] in TD | 4.9 | 6.0 | 4.2 | 4.2 | 4.1 | 4.6 | 6.2 | 5.9 | 6.1 |
| | | Stress [N/mm$^2$] at 10% Elongation in MD | 1.45 | 2.03 | 2.15 | 1.59 | 2.66 | 4.50 | 2.41 | 2.39 | 2.82 |
| | | Stress [N/mm$^2$] at 50% Elongation in TD | 0.59 | 0.57 | 0.69 | 0.62 | 0.64 | 0.69 | 0.67 | 0.70 | 0.73 |

[Table 2]

| | | | Example 10 | Example 11 | Example 12 | Example 13 | Example 14 | Example 15 | Example 16 | Example 17 | Example 18 |
|---|---|---|---|---|---|---|---|---|---|---|---|
| Surface Layer | Blend Ratio (parts by mass) | PP1 | 50 | 50 | 50 | 50 | 50 | 50 | 50 | 50 | 50 |
| | | Inorganic Filler | 50 | 50 | 50 | 50 | 50 | 50 | 50 | 50 | 50 |
| Elastomer Layer (Core Layer) | Blend Ratio (parts by mass) | Propylene-Based Elastomer | 100 | 100 | 100 | 100 | 100 | 100 | 100 | 100 | 100 |
| Elastomer Layer (Intermediate Layer) | Blend Ratio (parts by mass) | SIS Elastomer 1 | - | - | 30 | 50 | 30 | - | 50 | - | 30 |
| | | SIS Elastomer 2 | - | - | - | - | - | 50 | - | 30 | - |
| | | SIS Elastomer 3 | 100 | 100 | 70 | 50 | 70 | 50 | 50 | 70 | 70 |
| Thickness [μm] of Raw Film | First Surface Layer | | 1.264 | 3.5 | 1.37 | 2.15 | 1.58 | 3.5 | 3.5 | 3.5 | 3.5 |
| | Elastomer Layer(First Intermediate Layer) | | 8.84 | 7.0 | 9.06 | 8.99 | 8.35 | 8.4 | 8.4 | 7.0 | 7.0 |
| | Elastomer Layer (Core Layer) | | 15.54 | 14.0 | 13.31 | 12.51 | 15.70 | 11.2 | 11.2 | 14.0 | 14.0 |
| | Elastomer Layer (Second Intermediate Layer) | | 8.09 | 7.0 | 9.88 | 9.39 | 7.92 | 8.4 | 8.4 | 7.0 | 7.0 |
| | Second Surface Layer | | 1.264 | 3.5 | 1.37 | 1.96 | 1.44 | 3.5 | 3.5 | 3.5 | 3.5 |
| | Entire Thickness | | 35.0 | 35.0 | 35.0 | 35.0 | 35.0 | 35.0 | 35.0 | 35.0 | 35.0 |
| | Thickness of Surface Layer: Thickness of Elastomer Layer | | 1 : 25.7 | 1:8 | 1 : 23.5 | 1 : 14.4 1 : 15.8 | 1 : 20.2 1: 22.2 | 1 : 8 | 1 : 8 | 1 : 8 | 1 : 8 |
| Styrene Unit Content Rate [%] in Elastomer Layer | | | 9.4 | 9.0 | 15.9 | 19.6 | 13.7 | 15.9 | 19.8 | 11.6 | 13.5 |
| Shore A Hardness [-] of Elastomer Layer | | | 55.5 | 56.0 | 57.6 | 59.9 | 58.9 | 56.8 | 59.8 | 57.5 | 59.0 |
| Content Rate [%] of Olefin-Based Elastomer in Entire Stretchable Film | | | 44.4 | 40.0 | 38.0 | 35.8 | 44.9 | 32.0 | 32.0 | 40.0 | 40.0 |
| Content Rate [%] of Styrene-Based Elastomer in Entire Stretchable Film | | | 48.4 | 40.0 | 54.1 | 52.5 | 46.5 | 48.0 | 48.0 | 40.0 | 40.0 |

(continued)

| Evaluations | | Example 10 | Example 11 | Example 12 | Example 13 | Example 14 | Example 15 | Example 16 | Example 17 | Example 18 |
|---|---|---|---|---|---|---|---|---|---|---|
| | Hysteresis Loss [%] | 31.8 | 34.8 | 31.4 | 35.3 | 31.7 | 33.2 | 35.7 | 35.5 | 34.7 |
| | Permanent Strain [%] in TD | 6.7 | 6.4 | 6.1 | 6.2 | 6.2 | 6.8 | 6.0 | 8.1 | 6.4 |
| | Stress [N/mm$^2$] at 10% Elongation in MD | 1.25 | 1.65 | 2.33 | 4.07 | 1.96 | 1.67 | 2.69 | 1.92 | 1.97 |
| | Stress [N/mm$^2$] at 50% Elongation in TD | 1.14 | 0.99 | 0.88 | 0.94 | 1.06 | 0.97 | 1.08 | 1.05 | 1.03 |

[Table 3]

| | | | Example 19 | Example 20 | Example 21 | Example 22 | Example 23 | Example 24 | Example 25 |
|---|---|---|---|---|---|---|---|---|---|
| Surface Layer | Blend Ratio (parts by mass) | PP2 | 50 | - | - | - | - | - | - |
| | | PP3 | - | 50 | - | - | - | - | - |
| | | PP4 | - | - | 50 | - | - | - | - |
| | | PP5 | - | - | - | 50 | - | - | - |
| | | PP6 | - | - | - | - | 50 | - | - |
| | | PP7 | - | - | - | - | - | 50 | - |
| | | PP8 | - | - | - | - | - | - | 50 |
| | | Inorganic Filler | 50 | 50 | 50 | 50 | 50 | 50 | 50 |
| Elastomer Layer (Core Layer) | Blend Ratio (parts by mass) | Propylene-Based Elastomer | 100 | 100 | 100 | 100 | 100 | 100 | 100 |
| Elastomer Layer (Intermediate Layer) | Blend Ratio (parts by mass) | SIS Elastomer 1 | 30 | 30 | 30 | 30 | 30 | 30 | 30 |
| | | SIS Elastomer 3 | 70 | 70 | 70 | 70 | 70 | 70 | 70 |
| Thickness [μm] of Raw Film | | First Surface Layer | 3.5 | 3.5 | 3.5 | 3.5 | 3.5 | 3.5 | 3.5 |
| | | Elastomer Layer (First Intermediate Layer) | 7.0 | 7.0 | 7.0 | 7.0 | 7.0 | 7.0 | 7.0 |
| | | Elastomer Layer (Core Layer) | 14.0 | 14.0 | 14.0 | 14.0 | 14.0 | 14.0 | 14.0 |
| | | Elastomer Layer (Second Intermediate Layer) | 7.0 | 7.0 | 7.0 | 7.0 | 7.0 | 7.0 | 7.0 |
| | | Second Surface Layer | 3.5 | 3.5 | 3.5 | 3.5 | 3.5 | 3.5 | 3.5 |
| | | Entire Thickness | 35.0 | 35.0 | 35.0 | 35.0 | 35.0 | 35.0 | 35.0 |
| | | Thickness of Surface Layer: Thickness of Elastomer Layer | 1 : 8 | 1 : 8 | 1 : 8 | 1 : 8 | 1 : 8 | 1 : 8 | 1 : 8 |
| Styrene Unit Content Rate [%] in Elastomer Layer | | | 13.5 | 13.5 | 13.5 | 13.5 | 13.5 | 13.5 | 13.5 |

EP 4 667 213 A1

(continued)

| | Example 19 | Example 20 | Example 21 | Example 22 | Example 23 | Example 24 | Example 25 |
|---|---|---|---|---|---|---|---|
| Shore A Hardness [-] of Elastomer Layer | 59.0 | 59.0 | 59.0 | 59.0 | 59.0 | 59.0 | 59.0 |
| Content Rate [%] of Olefin-Based Elastomer in Entire Stretchable Film | 40.0 | 40.0 | 40.0 | 40.0 | 40.0 | 40.0 | 40.0 |
| Content Rate [%] of Styrene-Based Elastomer in Entire Stretchable Film | 40.0 | 40.0 | 40.0 | 40.0 | 40.0 | 40.0 | 40.0 |
| Evaluations — Hysteresis Loss [%] | 34.0 | 34.7 | 35.0 | 35.3 | 35.2 | 35.9 | 35.6 |
| Evaluations — Permanent Strain [%] in TD | 6.2 | 6.4 | 6.4 | 6.5 | 6.4 | 6.6 | 6.5 |
| Evaluations — Stress [N/mm$^2$] at 10% Elongation in MD | 1.90 | 1.80 | 1.70 | 1.90 | 2.08 | 1.88 | 1.71 |
| Evaluations — Stress [N/mm$^2$] at 50% Elongation in TD | 1.04 | 1.02 | 1.00 | 0.96 | 1.04 | 1.08 | 0.97 |

[Table 4]

| | | | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 | Comparative Example 11 | Comparative Example 12 | Comparative Example 13 |
|---|---|---|---|---|---|---|---|---|---|---|---|---|---|---|---|
| Surface Layer | Blend Ratio (parts by mass) | PP1 | 50 | 50 | 50 | 50 | 50 | 50 | 50 | 50 | 50 | 50 | 50 | 50 | 50 |
| | | Inorganic Filler | 50 | 50 | 50 | 50 | 50 | 50 | 50 | 50 | 50 | 50 | 50 | 50 | 50 |
| Elastomer Layer | Blend Ratio (parts by mass) | Propylene-Based Elastomer | 100 | 80 | - | - | - | - | - | - | - | - | - | - | - |
| | | Ethylene-Based Elastomer | - | - | 80 | - | - | - | - | - | - | - | - | - | - |
| | | SIS Elastomer 1 | - | - | - | 80 | - | - | 100 | 40 | 60 | 40 | 60 | 80 | - |
| | | SIS Elastomer 2 | - | - | - | - | 80 | - | - | - | - | - | - | 20 | - |
| | | SIS Elastomer 7 | - | - | - | - | - | - | - | 60 | 40 | - | - | - | - |
| | | SIS Elastomer 8 | - | - | - | - | - | - | - | - | - | 60 | 40 | - | - |
| | | SBS Elastomer | - | - | - | - | | 80 | - | - | - | - | - | - | 100 |
| | | SEBS Elastomer | - | 20 | 20 | 20 | 20 | 20 | - | - | - | - | - | - | - |

(continued)

| | | | Compara-tive Example 1 | Compara-tive Example 2 | Compara-tive Example 3 | Compara-tive Example 4 | Compara-tive Example 5 | Compara-tive Example 6 | Compara-tive Example 7 | Compar-ative Example 8 | Compara-tive Example 9 | Compara-tive Example 10 | Compara-tive Example 11 | Compara-tive Example 12 | Compara-tive Example 13 |
|---|---|---|---|---|---|---|---|---|---|---|---|---|---|---|---|
| Surface Layer | Blend Ratio (parts by mass) | PP1 | 50 | 50 | 50 | 50 | 50 | 50 | 50 | 50 | 50 | 50 | 50 | 50 | 50 |
| | | Inorganic Filler | 50 | 50 | 50 | 50 | 50 | 50 | 50 | 50 | 50 | 50 | 50 | 50 | 50 |
| Thickness [μm] of Raw Film | First Surface Layer | | 2.32 | 1.69 | 2.87 | 3.5 | 3.5 | 3.5 | 3.5 | 3.5 | 3.5 | 3.5 | 3.5 | 3.5 | 3.5 |
| | Elastomer Layer | | 30.4 | 31.6 | 29.3 | 28.0 | 28.0 | 28.0 | 28.0 | 28.0 | 28.0 | 28.0 | 28.0 | 28.0 | 28.0 |
| | Second Surface Layer | | 2.32 | 1.69 | 2.87 | 3.5 | 3.5 | 3.5 | 3.5 | 3.5 | 3.5 | 3.5 | 3.5 | 3.5 | 3.5 |
| | Entire Thickness | | 35.0 | 35.0 | 35.0 | 35.0 | 35.0 | 35.0 | 35.0 | 35.0 | 35.0 | 35.0 | 35.0 | 35.0 | 35.0 |
| | Thickness of Surface Layer: Thickness of Elastomer Layer | | 1 : 13.1 | 1 : 18.7 | 1 : 10.2 | 1 : 8 | 1:8 | 1: 8 | 1 : 8 | 1 : 8 | 1 : 8 | 1 : 8 | 1 : 8 | 1 : 8 | 1 : 8 |
| Styrene Unit Content Rate [%] in Elastomer Layer | | | 0 | 2.4 | 2.4 | 41.2 | 30.4 | 34.4 | 48.0 | 28.2 | 34.8 | 31.8 | 37.2 | 45.4 | 40.0 |
| Shore A Hardness [-] of Elastomer Layer | | | 67.0 | 62.0 | 59.6 | 60.4 | 52.4 | 76.4 | 65.0 | 45.2 | 51.8 | 49.4 | 54.6 | 63.0 | 85.0 |
| Content Rate [%] of Olefin-Based Elastomer in Entire Stretchable Film | | | 86.8 | 72.3 | 66.9 | - | - | - | - | - | - | - | - | - | - |
| Content Rate [%] of Styrene-Based Elastomer in Entire Stretchable Film | | | 0 | 18.1 | 16.7 | 80.0 | 80.0 | 80.0 | 80.0 | 80.0 | 80.0 | 80.0 | 80.0 | 80.0 | 80.0 |

(continued)

| | | | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 | Comparative Example 11 | Comparative Example 12 | Comparative Example 13 |
|---|---|---|---|---|---|---|---|---|---|---|---|---|---|---|---|
| Surface Layer | Blend Ratio (parts by mass) | PP1 | 50 | 50 | 50 | 50 | 50 | 50 | 50 | 50 | 50 | 50 | 50 | 50 | 50 |
| | | Inorganic Filler | 50 | 50 | 50 | 50 | 50 | 50 | 50 | 50 | 50 | 50 | 50 | 50 | 50 |
| Evaluations | Hysteresis Loss [%] | | 39.9 | 38.1 | 44.9 | 49.7 | 41.8 | 59.5 | 60.1 | 38.0 | 40.6 | 43.4 | 48.2 | 50.5 | 66.0 |
| | Permanent Strain [%] in TD | | 7.4 | 7.3 | 8.3 | 9.2 | 7.7 | 11.1 | 11.2 | 7.0 | 7.5 | 8.0 | 8.9 | 9.3 | 12.3 |
| | Stress [N/mm$^2$] at 10% Elongation in MD | | 1.10 | 1.16 | 0.74 | 4.37 | 2.58 | 3.41 | 7.52 | 2.77 | 3.80 | 3.12 | 4.13 | 5.19 | 3.91 |
| | Stress [N/mm$^2$] at 50% Elongation in TD | | 1.46 | 1.20 | 1.05 | 1.04 | 0.84 | 1.20 | 1.61 | 0.68 | 0.85 | 0.78 | 0.99 | 1.21 | 1.36 |

EP 4 667 213 A1

22

[Table 5]

| | | | Comparative Example 14 | Comparative Exanple 15 | Comparative Example 16 | Comparative Example 17 | Comparative Exanple 18 | Comparative Example 19 | Comparative Example 20 | Comparative Example 21 | Comparative Example 22 | Comparative Example 23 | Comparative Exanple 24 | Comparative Example 25 |
|---|---|---|---|---|---|---|---|---|---|---|---|---|---|---|
| Surface Layer | Blend Ratio (parts by mass) | PP1 | 50 | 50 | 50 | 50 | 50 | 50 | 50 | 50 | 50 | 50 | 50 | 50 |
| | | Inorganic Filler | 50 | 50 | 50 | 50 | 50 | 50 | 50 | 50 | 30 | 50 | 50 | 50 |
| Elastomer Layer (Core Layer) | Blend Ratio (parts by mass) | Propylene-Based Elastomer | - | - | - | - | - | - | 100 | 100 | 100 | 100 | 100 | 100 |
| | | SIS Elastomer 1 | 100 | 100 | 100 | 100 | 100 | 100 | - | - | - | - | - | - |
| Elastomer Layer (Intermediate Layer) | Blend Ratio (parts by masss) | SIS Elastomer 1 | - | - | - | - | - | - | 100 | - | 50 | - | - | 50 |
| | | SIS Elastomer 2 | - | - | - | - | - | - | - | 100 | - | 50 | 50 | - |
| | | SIS Elastomer 3 | - | - | - | - | - | - | - | - | 50 | 50 | 50 | 50 |
| | | SIS Elastomer 5 | 100 | 100 | - | - | - | - | - | - | - | - | - | - |
| | | SIS Elastomer 6 | - | - | - | - | 100 | 100 | - | - | - | - | - | - |
| | | SIS Elastomer 7 | - | - | 100 | 100 | - | - | - | - | - | - | - | - |

23

| | | Comparative Example 14 | Comparative Example 15 | Comparative Example 16 | Comparative Example 17 | Comparative Exanple 18 | Comparative Example 19 | Comparative Example 20 | Comparative Example 21 | Comparative Example 22 | Comparative Example 23 | Comparative Exanple 24 | Comparative Example 25 |
|---|---|---|---|---|---|---|---|---|---|---|---|---|---|
| Thickness [μm] of Raw Film | First Surface Layer | 3.5 | 3.5 | 3.5 | 3.5 | 3.5 | 3.5 | 3.5 | 3.5 | 3.5 | 3.5 | 3.5 | 3.5 |
| | Elastomer Layer (First Intermediate Layer) | 5.6 | 4.2 | 8.4 | 5.6 | 9.8 | 8.4 | 7.0 | 7.0 | 7.0 | 7.0 | 5.6 | 5.6 |
| | Elastomer Layer (Core Layer) | 16.8 | 19.6 | 11.2 | 16.8 | 8.4 | 11.2 | 14.0 | 14.0 | 14.0 | 14.0 | 16.8 | 16.8 |
| | Elastomer Layer (Second Internediate Layer) | 5.6 | 4.2 | 8.4 | 5.6 | 9.8 | 8.4 | 7.0 | 7.0 | 7.0 | 7.0 | 5.6 | 5.6 |
| | Second Surface Layer | 3.5 | 3.5 | 3.5 | 3.5 | 3.5 | 3.5 | 3.5 | 3.5 | 3.5 | 3.5 | 3.5 | 3.5 |
| | Entire Thickness | 35.0 | 35.0 | 35.0 | 35.0 | 35.0 | 35.0 | 35.0 | 35.0 | 35.0 | 35.0 | 35.0 | 35.0 |
| | Thickness of Surface Layer : Thickness of Elastomer Layer | 1:8 | 1:8 | 1:8 | 1:8 | 1:8 | 1:8 | 1:8 | 1:8 | 1:8 | 1:8 | 1:8 | 1:8 |
| Styrene Unit Content Rate [%] in Elastomer Layer | | 35.2 | 38.4 | 28.2 | 34.8 | 35.4 | 37.2 | 24.0 | 17.5 | 16.5 | 13.3 | 13.3 | 16.5 |
| Shore A Hardness [-] of Elastomer Layer | | 47.4 | 51.8 | 45.2 | 51.8 | 51.0 | 53.0 | 66.0 | 61.0 | 61.0 | 58.5 | 58.5 | 61.0 |
| Content Rate [%] of Ole fin-Based Elastomer in Entire Stretchable Film | | - | - | - | - | - | - | 40.0 | 40.0 | 40.0 | 40.0 | 48.0 | 48.0 |
| Content Rate [%] of Styrene-Based Elastomer in Entire Stretchable Film | | 80.0 | 80.0 | 80.0 | 80.0 | 80.0 | 80.0 | 40.0 | 40.0 | 40.0 | 40.0 | 32.0 | 32.0 |

EP 4 667 213 A1

24

(continued)

| Evaluations | Compara-tive Example 14 | Compara-tive Exanple 15 | Compara-tive Example 16 | Compara-tive Example 17 | Compara-tive Exanple 18 | Compara-tive Example 19 | Compara-tive Example 20 | Compara-tive Example 21 | Compara-tive Example 22 | Compara-tive Example 23 | Compara-tive Exanple 24 | Compara-tive Example 25 |
|---|---|---|---|---|---|---|---|---|---|---|---|---|
| Hysteresis Loss [%] | 50.2 | 51.1 | 45.0 | 48.0 | 50.5 | 51.7 | 48.0 | 42.0 | 37.0 | 37.1 | 36.9 | 37.0 |
| Permanent Strain [%] in TD | 9.3 | 10.5 | 7.7 | 8.9 | 8.3 | 9.6 | 8.6 | 7.2 | 6.4 | 7.2 | 7.4 | 7.2 |
| Stress [N/nm$^2$] at 10% Elongation in MD | 4.10 | 4.97 | 3.19 | 4.43 | 4.66 | 5.15 | 3.90 | 2.77 | 2.77 | 2.14 | 2.09 | 2.79 |
| Stress [N/nm$^2$] at 50% Elongation in TD | 0.96 | 1.06 | 0.88 | 0.98 | 0.94 | 1.26 | 1.62 | 1.20 | 1.18 | 1.09 | 1.11 | 1.17 |

As can be seen from Tables 1 to 3, the stretchable films of Examples 1 to 25 each include an elastomer layer that contains a styrene-based elastomer, and has a hysteresis loss of less than 36%, resulting in excellent stretchability.

In contrast, as can be seen from Table 4, the stretchable films of Comparative Examples 1 to 3 each include a single elastomer layer, contain an olefin-based elastomer (a propylene-based elastomer, an ethylene-based elastomer), and contain no SIS elastomer, resulting in poor stretchability (the hysteresis loss of 36% or more). Further, the stretchable films of Comparative Examples 1 and 2 each have the Shore A hardness of 60 or more, resulting in poor stretchability (the hysteresis loss of 36% or more).

In the stretchable films of Comparative Examples 4, 7, 12, and 13, a styrene unit content rate relative to the entire elastomer layer is larger than 35 mass%, and the Shore A hardness is 60 or more, resulting in poor stretchability (the hysteresis loss of 36% or more).

In the stretchable film of Comparative Example 5, the SIS elastomer and the SEBS elastomer had poor compatibility, and a large number of aggregates (foreign matter) were generated in the film due to the aggregation of the added SEBS component, resulting in poor stretchability (the hysteresis loss of 36% or more).

In the stretchable film of Comparative Example 6, the elastomer layer contains an SBS elastomer having a very high Shore A hardness and has a Shore A hardness of 60 or more, resulting in poor stretchability (the hysteresis loss of 36% or more).

In the stretchable films of Comparative Examples 8 to 10, 16, and 17, plastic deformation significantly proceeds in the SIS elastomer 7 or the SIS elastomer 8 contained in the elastomer layer, and the permanent strain in the TD increases, resulting in poor stretchability (the hysteresis loss of 36% or more). One possible reason for this is that the high ratio of diblock components in the styrene-based elastomer resulted in a reduced recovery force after the elongation.

In the stretchable films of Comparative Examples 11, 14, 15, 18, and 19, the styrene unit content rate relative to the entire elastomer layer is larger than 35 mass%, resulting in poor stretchability (the hysteresis loss of 36% or more).

In the stretchable films of Comparative Examples 20 and 21, the SIS elastomer 1 or the SIS elastomer 2 contained alone in the core layer of the elastomer layer each have a styrene unit content rate of 30 mass% or more, resulting in poor stretchability (the hysteresis loss of 36% or more).

In the stretchable films of Comparative Examples 22 to 25, the intermediate layer of the elastomer layer contains 50% or more of the SIS elastomer 1 or the SIS elastomer 2 having a styrene unit content rate of 30 mass% or more. In this case, when, as in Comparative Examples 22 to 25, the ratio of the total thickness of the intermediate layer to the total (28 μm, which is the thickness of the entire elastomer layer) of the thickness of the core layer of the elastomer layer and the total thickness of the intermediate layers becomes 50% or less, the stress at 50% elongation in the TD improves, but the decrease in stretchability cannot be suppressed, resulting in poor stretchability (the hysteresis loss of 36% or more). Further, it is found that in the stretchable films of Comparative Examples 22 and 25, the Shore A hardness is 60 or more, resulting in poor stretchability (the hysteresis loss of 36% or more).

INDUSTRIAL APPLICABILITY

As can be seen from above, the present invention is suitable for a stretchable film used for clothes such as underwear, a waistband of a paper diaper, a side panel, a leg gather, an incontinence article, a sanitary napkin, a bandage, a surgical drape, a fastening band, a hat, swimming pants, a sports supporter, a medical supporter, an adhesive plaster, and the like.

DESCRIPTION OF REFERENCE CHARACTERS

| 1 | Stretchable Film |
| 5 | Elastomer Layer |
| 6, 7 | Surface Layer |
| 8 | Core Layer |
| 9, 10 | Intermediate Layer |
| 11 | Elastomer Layer |
| 20 | Stretchable Film |

**Claims**

1.  A stretchable film comprising:

    an elastomer layer; and

a surface layer stacked on at least one surface of the elastomer layer,

the elastomer layer containing a styrene-based elastomer,

the surface layer being made of an olefin-based resin composition containing an olefin-based resin and an inorganic filler,

the stretchable film having a hysteresis loss of less than 36%, determined as follows:

(Hysteresis Loss)

a strip-shaped test piece is cut from the stretchable film, the test piece having 100 mm in one direction of the film and 25 mm in a direction orthogonal to the one direction, the test piece is then fixed to grippers of a tester so that a distance between the grippers is 25 mm, and the test piece is then elongated in its longitudinal direction at a speed 254 mm/min so that a strain (elongation) calculated by the following equation (1) is 100%, an integral area in a stress-strain curve (S-S curve) during the elongation is defined as S1, an integral area in a stress-strain curve (S-S curve) during contraction of the test piece at the same speed as for the elongation, immediately after the elongation so that s load (N/25 mm) of the test piece reaches 0 is defined as S2, and the hysteresis loss [%] is calculated from the following equation (2),

$$\text{Strain } [\%] = (L1 - L0)/L0 \times 100 \ (1)$$

$$\text{Hysteresis loss } [\%] = S/S1 \times 100 \ (2)$$

where L0 is a distance (mm) between the grippers before the elongation; L1 is a distance (mm) between the grippers after the elongation; S1 is an integral area in a stress-strain curve during elongation from 0% to 100% strain; S2 is an integral area in a stress-strain curve during contraction from 100% strain to 0 stress; and S is an integral area (S1 - S2) in a stress-strain curve obtained by subtracting the integral area S2 from the integral area S1.

2. The stretchable film of claim 1, wherein the styrene-based elastomer is a styrene-isoprene-styrene copolymer (SIS elastomer).

3. The stretchable film of claim 2, wherein

the elastomer layer includes: a core layer; and an intermediate layer stacked on at least one surface of the core layer, and

the intermediate layer is provided between the core layer and the surface layer.

4. The stretchable film of claim 3, wherein

the core layer is made of an olefin-based elastomer, and

the intermediate layer is made of the styrene-isoprene-styrene copolymer (SIS elastomer).

5. The stretchable film of claim 4, wherein the olefin-based elastomer is made of a propylene-based elastomer.

6. The stretchable film of any one of claims 1 to 5, wherein the olefin-based resin is random polypropylene, and the inorganic filler is calcium carbonate.

# FIG.1

# FIG.2

# FIG.3

# FIG.4

# FIG.5

# FIG.6

EP 4 667 213 A1

# FIG.7

31

# EP 4 667 213 A1

## INTERNATIONAL SEARCH REPORT

International application No.

**PCT/JP2024/006828**

### A. CLASSIFICATION OF SUBJECT MATTER

*B32B 7/022*(2019.01)i; *A61F 13/02*(2024.01)i; *A61F 13/15*(2006.01)i; *A61F 13/49*(2006.01)i; *B32B 27/20*(2006.01)i; *B32B 27/32*(2006.01)i; *C08J 7/04*(2020.01)i
FI: B32B7/022; A61F13/02 310D; A61F13/15 100; A61F13/49 319; B32B27/20; B32B27/32; C08J7/04 Z CEQ

According to International Patent Classification (IPC) or to both national classification and IPC

### B. FIELDS SEARCHED

Minimum documentation searched (classification system followed by classification symbols)

B32B7/022; A61F13/02; A61F13/15; A61F13/49; B32B27/20; B32B27/32; C08J7/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2021-123677 A (C.I.TAKIRON CORP.) 30 August 2021 (2021-08-30) | 1-3, 6 |
| | claims, paragraphs [0014], [0028], examples | |
| Y | | 1-2 |
| A | | 4-5 |
| Y | WO 2017/034030 A1 (3M INNOVATIVE PROPERTIES COMPANY) 02 March 2017 (2017-03-02) | 1-2 |
| | examples | |
| A | | 3-6 |
| Y | JP 2016-112878 A (NITTO DENKO CORPORATION) 23 June 2016 (2016-06-23) | 1-2 |
| | paragraphs [0062], [0066] | |
| A | | 3-6 |
| A | JP 2020-183119 A (NITTO DENKO CORPORATION) 12 November 2020 (2020-11-12) | 1-6 |
| | entire text | |

☑ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **10 April 2024** | **23 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

32

**INTERNATIONAL SEARCH REPORT**

| International application No. |
| --- |
| **PCT/JP2024/006828** |

### C.     DOCUMENTS CONSIDERED TO BE RELEVANT

| Category* | Citation of document, with indication, where appropriate, of the relevant passages | Relevant to claim No. |
| --- | --- | --- |
| A | WO 2016/013577 A1 (C I KASEI CO., LTD.) 28 January 2016 (2016-01-28)<br>    entire text | 1-6 |

Form PCT/ISA/210 (second sheet) (July 2022)

## INTERNATIONAL SEARCH REPORT
### Information on patent family members

International application No.

**PCT/JP2024/006828**

| Patent document cited in search report | | | Publication date (day/month/year) | Patent family member(s) | | | Publication date (day/month/year) |
|---|---|---|---|---|---|---|---|
| JP | 2021-123677 | A | 30 August 2021 | (Family: none) | | | |
| WO | 2017/034030 | A1 | 02 March 2017 | KR 10-2018-0044299 | | A | |
| | | | | CN 108136743 | | A | |
| JP | 2016-112878 | A | 23 June 2016 | US 2017/0320304 paragraph [0085] | | A1 | |
| | | | | EP 3231604 | | A1 | |
| | | | | CN 107000391 | | A | |
| | | | | KR 10-2017-0094155 | | A | |
| JP | 2020-183119 | A | 12 November 2020 | US 2022/0212451 entire text | | A1 | |
| | | | | EP 3960442 | | A1 | |
| WO | 2016/013577 | A1 | 28 January 2016 | US 2017/0203554 | | A1 | |
| | | | | EP 3173228 | | A1 | |
| | | | | CN 106536191 | | A | |

Form PCT/ISA/210 (patent family annex) (July 2022)

34

## EP 4 667 213 A1

**Patent documents cited in the description**

- JP 2016112878 A